# EUROPÄISCHE PATENTSCHRIFT

(11) **EP 0 705 608 B1**
(45) Veröffentlichungstag und Bekanntmachung des Hinweises auf die Patenterteilung: **03.02.1999**
(21) Anmeldenummer: 94890161.6
(22) Anmeldetag: 05.10.1994
(51) Int. Cl.: A61L 2/12, A61L 2/24

(54) **Anlage zum Sterilisieren und/oder Desinfizieren pump- oder rieselfähiger Medien**
Installation for sterilizing and/or disinfecting pumpable or free-flowing matter
Installation pour stériliser et/ou désinfecter des matières pompables ou coulables

(43) Veröffentlichungstag der Anmeldung: 10.04.1996
(73) Patentinhaber: Katschnig, Helmut, Dr., A-8750 Judenburg Steiermark (AT)
(72) Erfinder: Katschnig, Helmut, Dr., A-8750 Judenburg (AT); Stegmüller, Wolfgang, Ing., A-8755 St. Peter/Judenburg (AT); Gruber, Ernst, A-8750 Judenburg (AT)
(74) Vertreter: Itze, Peter, Dipl.-Ing.

(56) Entgegenhaltungen:
- EP-A- 0 408 946
- WO-A-86/02842
- DE-A- 3 430 673

## Beschreibung

Die Erfindung bezieht sich auf eine Anlage zum Sterilisieren, Pasteurisieren und/oder Desinfizieren pump- oder rieselfähiger Medien, in welcher zum Erhitzen ein Mikrowellengerät vorgesehen ist, durch welches das zu behandelnde Medium hindurchgefördert wird.

Aus der EP-A-0116921 geht eine Einrichtung hervor, welche zum Sterilisieren von Kupplungen und durch diese hindurchgeführte Flüssigkeiten dient, wobei es sich bei diesen Kupplungen um medizinische Verbindungen zwischen extrakorporalen und intrakorporalen Einrichtungen handelt, und zwar beispielsweise um Einrichtungen zur Perititeronaldialyse. Eine Drucksterilisation ist bei dieser Ausführung nicht vorgesehen, sondern diese dient lediglich zur Sterilisation einer ersten Flüssigkeitscharge, um damit Infektionen im Körper zu verhindern.

Aus der DE-A-3430673 ist eine Einrichtung zum Pasteurisieren und Sterilisieren von rieselfähigen Materialien vorgesehen, bei welchen rieselfähiges oder stückiges Gut durch einen Hohlleiter mittels einen Förderraum oben und unten begrenzenden Förderbändern hindurchgeführt wird, in welchem das Gut durch Mikrowellen bestrahlt wird, um darin vorhandene Bakterien, Pilzkeime od. dgl. abzutöten. Auch diese Einrichtung arbeitet unter Normaldruck.

Aus der US-A-4,417,116 geht eine Einrichtung zur Erwärmung von Wasser hervor, welche als Durchlauferhitzer ausgebildet ist. Dazu ist in einer Mikrowellenkammer ein quaderförmiger Körper vorgesehen, der in Längsrichtung durchgehende Bohrungen aufweist, durch welche das Wasser hindurchgeführt wird. All diese Bohrungen enden auf jeder Seite in einer gemeinsamen Kammer, wobei durch die eine Kammer Wasser eingeführt und durch die andere Kammer das Wasser abgeleitet wird. Diese Ausführung arbeitet ebenso bei Normaldruck, wobei für die Steuerung des Magnetrons die Temperatur des aus dem Gerät austretenden Wassers gemessen ist. Wird ein bestimmter Grenzwert überschritten, dann wird die Energiezufuhr zum Magnetron abgeschaltet.

Der Erfindung liegt nun die Aufgabe zugrunde eine Anlage der eingangs genannten Art zu schaffen, mit welcher auch eine Drucksterilisation möglich ist, und zwar im kontinuierlichen Durchlaufverfahren.

Erfindungsgemäß wird diese Aufgabe dadurch gelöst, daß im Strömungsweg zwei zwangsfördernde Förderaggregate vorgesehen sind, von denen eines vor dem Mikrowellengerät und eines nach demselben angeordnet ist, wobei zwischen den beiden Förderaggregaten ein Drucksensor eingebaut ist, der den in der Leitung zwischen den beiden Förderaggregaten herrschenden Druck als Steuersignal an eine, die beiden Förderaggregate miteinander logisch verbindende Steuerung weiterleitet. Dadurch wird erreicht, daß über die zwei Förderaggregate ein Druck im System aufgebaut werden kann, welcher auch im Durchlaufverfahren durch ensprechende Steuerung des Antriebes der Förderaggregate aufrechterhalten werden kann. Auf diese Weise ist es möglich, im Durchlaufverfahren pump- oder rieselfähige Materialien hindurchzufördern, wobei für rieselfähige Materialien beispielsweise geschlossene Förderschnecken od. dgl. eingesetzt werden können.

Für die Desinfektion, Sterilisation oder Pasteurisation von pumpfähigen Medien können als zwangsfördernde Förderaggregate zwangsfördernde Pumpen, vorzugsweise Schlauchquetschpumpen, vorgesehen sein. Damit ist ein kontinuierlicher Medienfluß durch die Anlage sichergestellt, wobei über die Drehzahl der Pumpen bzw. deren Förderleistung der im System herrschende Druck gesteuert wird. Weiters kann dem Mikrowellengerät eine Temperaturhaltestrecke z.B. eine Rohrschlange nachgeschaltet sein, wobei zwischen Mikrowellengerät und Temperaturhaltestrecke ein Temperaturfühler angeordnet ist. Dadurch wird erzielt, daß die Behandlungskammer im Mikrowellengerät kleingehalten werden kann, weil die Wirkung der Temperatur dann auf der Temperaturhaltestrecke beibehalten wird, welche bereits außerhalb des Mikrowellengerätes diesem nachgeschaltet ist. Der vorgesehene Temperaturfühler dient dazu festzustellen, ob vor der Temperaturhaltestrecke die erforderliche Temperatur erreicht ist. Für eine noch genauere Kontrolle der Behandlungstemperatur kann am Ende der Temperaturhaltestelle ein zweiter Temperaturfühler angeordnet sein, wodurch festgestellt werden kann, ob über die gesamte Temperaturhaltestrecke die erwünschte Temperatur vorgelegen ist.

Bei einer besonders vorteilhaften Ausführung kann der Temperatursensor und der bzw. die Temperaturfühler mit einer zentralen Steuereinheit verbunden sein, über welche die Leistung der beiden zwangsfördernden Förderaggregate und gegebenenfalls des Magnetrons des Mikrowellengerätes steuerbar ist. Es wird damit die Durchflußgeschwindigkeit des Mediums anhand der ermittelten Parameter gesteuert, um solcherart zu gewährleisten, daß die gewünschte kombinierte Druck-Temperaturbehandlung des zu behandelnden Gutes ordnungsgemäß erreicht ist.

Das zu behandelnde Medium kann vor dem Mikrowellengerät über einen oder mehrere Wärmeaustauscher geführt sein, in welchen das bereits behandelte Medium mit dem zu behandelten Medium im Gegenstrom geführt ist. Dadurch wird einerseits eine raschere Abkühlung des behandelten Mediums und anderseits eine Vorwärmung des zu behandelnden Mediums erzielt, wodurch erhebliche Energiemengen eingespart sind. Aus gleichem Grunde kann bei Einsatz eines wassergekühlten Magnetrons das Kühlwasser durch einen weiteren Wärmetauscher geführt sein, in welchen das Kühlwasser gleichfalls im Gegenstrom mit dem zu behandelnden Medium geführt ist.

Um eine Rückverkeimung, also ein nachträgliches Infizieren von bereits entkeimtem Medium zu verhindern, kann innerhalb der Förderstrecke ein Abbruchgefäß angeordnet sein, und zwar vorzugsweise innerhalb des Mikrowellengerätes. Unter Abbruchgefäßen ist ein solches Gefäß zu verstehen, bei welchem der direkte Durchfluß vom Medium unterbrochen ist, und zwar dadurch, daß dem Gefäß von ober her zu behandelndes Medium zugeführt wird, welches durch einen Luftpolster hindurchfließt und dann das Gut von einem im Unterteil des Behälters sich ansammelnden Polster abgezogen wird. Die Anordnung innerhalb des Mikrowellengerätes hat den Vorteil, daß dieser Polster vor der Weiterleitung aus dem Mikrowellengerät nochmals aufgeheizt wird, und somit immer keimfreies Material aus dem Mikrowellengerät austritt. In besonders bevorzugter Weise kann das Abbruchgefäß direkt das Behandlungsgefäß für das pumpfähige Medium in dem Mikrowellengerät bilden.

In der Zeichnung ist ein Ausführungsbeispiel des Erfindungsgegenstandes dargestellt.

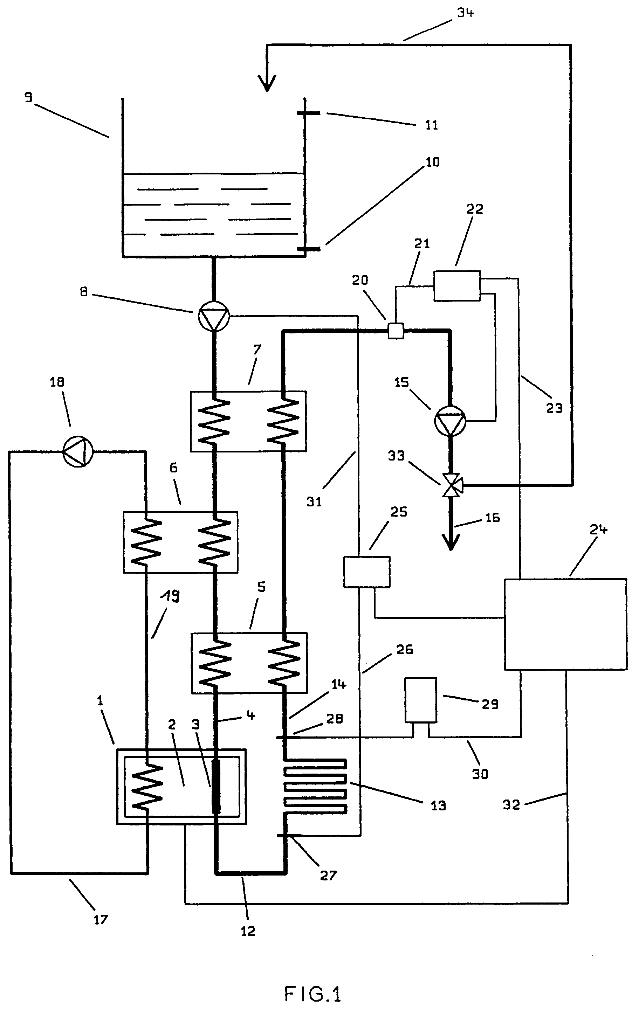

Figur 1 zeigt schematisch den Leitungsplan der erfindungsgemäßen Anlage.
Figur 2 zeigt im Detail die Behandlungskammer des Mikrowellengerätes.

Mit 1 ist ein Mikrowellengerät bezeichnet, das ein wassergekühltes Magnetron 2 aufweist, in dem sich eine Behandlungskammer 3 befindet. In diese Behandlungskammer mündet eine Leitung 4 ein, welche eine Wärmertauscherkaskade 5, 6, 7 durchläuft. In diese Leitung 4 wird über eine zwangsfördernde Pumpe, in vorliegendem Fall eine Schlauchquetschpumpe 8, zu behandelndes Gut aus einem Vorratsbehälter 9 hineingefördert. In dem Vorratsbehälter 9 sind zwei Niveausonden 10, 11 vorgesehen, welche das Ein-bzw. Ausschalten der Gesamtanlage bewirken. Das behandelte Gut wird über die Leitung 12 in eine Temperaturhaltestrecke 13 gebracht, welche in vorliegendem Fall aus einer Rohrschlange gebildet ist. Nach der Temperaturhaltestrecke 13 ist eine Rohrleitung 14 vorgesehen, welche den Wärmetauscher 5 und 7 durchströmt und zu einer zweiten zwangsfördernden Pumpe, gleichfalls eine Schlauchquetschpumpe 15 führt. Von der zwangsfördernden Pumpe 15 führt eine Leitung 16 zum Sammelkanal.

Das aus der Kühlung des wassergekühlten Magnetrons 2 über eine Leitung 17 abfließende Wasser wird über eine Umwälzpumpe 18 dem Wärmetauscher 6 zugeführt und von diesem über die Leitung 19 wieder zum Magnetron 2 rückgeleitet.

In der Leitung zwischen der Behandlungskammer 3 und der zweiten zwangsfördernden Pumpe 15 ist ein Drucksensor 20 eingebaut, der über eine Leitung 21 mit einer Drucksteuerung 22 verbunden ist, die ihrerseits über eine Leitung 23 mit einem zentralen Steuergerät 24 in Verbindung ist. Das zentrale Steuergerät erhält zusätzlich noch Daten über eine Temperatursteuerung 25, die ihrerseits über eine Leitung 26 mit einem Temperatursensor 27 verbunden ist, welcher zu Beginn der Temperaturhaltestrecke 13 angeordnet ist. Ein zweiter Temperatursensor 28 ist am Ende der Temperaturhaltestrecke 13 vorgesehen und steht über eine weitere Temperatursteuerung 29 und eine Leitung 30 wieder mit dem Steuergerät 24 in Verbindung. Von der Temperatursteuerung 25 ist eine Steuerleitung 31 vorgesehen, welche zur ersten zwangsfördernden Pumpe 8 führt. Vom Steuergerät 24 führt weiters noch eine Leitung 32 zur Stromanspeisung des Magnetrons 2.

Es können auch zwei Magnetrons vorgesehen sein, welche entweder hintereinandergeschaltet oder aber auch alterniv beschickbar sein können.

In der Leitung 16 ist der Zwangsförderpumpe 15 ein Umschaltventil 33 nachgeschaltet, welches mit einer Leitung 34 verbunden ist, die zum Vorratsbehälter 9 zurückführt.

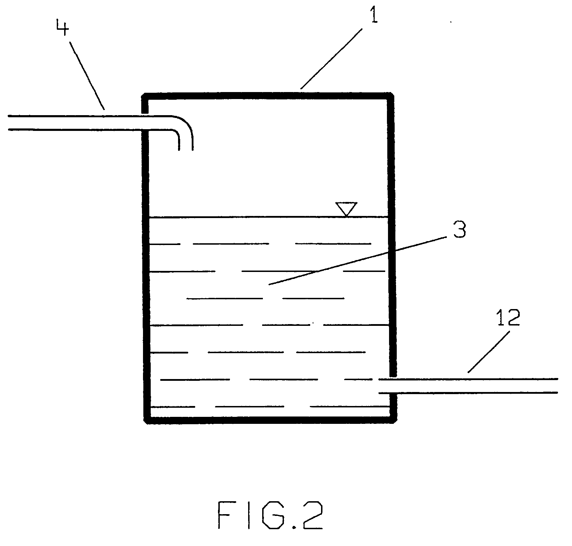

Wie aus Figur 2 ersichtlich, ist die Behandlungskammer 3 als sogenanntes Abbruchgefäß ausgebildet, das ist ein Gefäß, welches im vorliegenden Fall als Windkessel fungiert, da diese Anlage unter Überdruck arbeitet. Dieses Abbruchgefäß dient dazu, einen direkten Medienfluß zwischen der Leitung 4 und der Leitung 12 zu unterbinden, womit sichergestellt ist, daß bereits sterilisiertes Gut aus der Leitung 12 nicht mit noch nicht sterilisierten Gut aus der Leitung 4 in Verbindung kommt, wenn das Gerät in irgendwelchen Stadien abgeschaltet wird. Dazu führt die Leitung 4 am oberen Ende der Behandlungskammer 3 in diese hinein, wobei die Leitung sifonartig ausgebildet ist, um ein Ausrinnen von in der Leitung befindlichem Gut nach Abschalten der Pumpe 8 zu verhindern. Die zu behandelnde und mit Mikrowellen zu bestahlende Flüssigkeit sammelt sich am Boden des Gefäßes 3 an und wird nach entsprechender Aufheizung über die Leitung 12 aus der Mikrowellenbehandlungskammer herausgeführt.

Eine für rieselfähiges Gut ausgelegte Anlage ist analog aufgebaut, wobei anstelle von zwangsfördernden Pumpen zwangsfördernde Feststofförderer, wie z.B. geschlossene Förderschnecken, vorgesehen sind. Bei einer solchen Ausbildung könnte gegebenenfalls ein Abbruchgefäß entfallen, da ein Zurückfließen von Medium nur bedingt gegeben ist.

Die Funktion der erfindungsgemäßen Anlage wird nachstehend anhand eines zu sterilisierenden infektiösen Abwassers aus Spitälern beschrieben.

Das zu sterilisierende Abwasser wird im Vorratsbehälter 9 gesammelt, wobei die Anlage bei Erreichen des Niveaus der Niveausonde 11 eingeschaltet wird. Danach fördert die Pumpe 8 zu behandelndes Gut bei stillstehender Pumpe 15 durch die Wärmetauscher 7, 6 und 5 in die Behandlungskammer 3 des Magnetrons 2 des Mikrowellengerätes 1 und zwar solange bis der Drucksensor 20 den eingestellten Arbeitsdruck, vorliegend etwa 2 bar, anzeigt. Danach beginnt die zweite zwangsfördernde Pumpe zu arbeiten und zwar in der Weise, daß der Druck am Drucksensor 20 gleichgehalten wird. Die Kordination der Antriebe der beiden Pumpen erfolgt über das zentrale Steuergerät 24. Gleichzeitig wird über den Temperatursensor 27 ermittelt, ob die in der Behandlungskammer 3 befindliche Flüssigkeit auf einen vorgegebenen Wert, bei 2 bar etwa 120°C, erwärmt ist. Die gewünschte Erwärmung wird dadurch sichergestellt, daß die Förderleistung der Pumpe 8 zu Beginn sehr gering ist und erst gesteigert wird, wenn der angestrebteTemperaturwert erreicht ist. Ist dies der Fall, dann wird die Flüssigkeit durch die Temperaturhaltestrecke langsam hindurchgeführt, wobei der Temperaturabfall über den Temperatursensor 28 ermittelt und dem zentralen Steuergerät mitgeteilt wird. Ist der Temperaturabfall im vorgegebenen Rahmen, dann wird die Vorrichtung solange weiter betrieben, bis das Abwasser das Niveau der Niveausonde 10 erreicht und danach wird die Anlage abgeschaltet.

Die über die Leitung 4 dem Magnetron 2 zugeführte Flüssigkeit wird dabei in den Wärmetauschern 7, 6 und 5 dadurch vorgewärmt, daß in den Wärmetauschern 7 und 5 bereits behandelte, erhitzte Flüssigkeit mit der zu behandelnden kühlen Flüssigkeit im Gegenstrom geführt wird, und zwischen den Wärmetauschern 7 und 5 noch jener Wärmeaustauscher 6 angeordnet ist, der über das Kühlwasser des Magnetrons 2 gespeist ist. Es erfolgt damit eine kaskadenförmige Erwärmung der zu behandelnden Flüssigkeit, wobei gleichzeitig die behandelte Flüssigkeit auf die gewünschte Temperatur abgekühlt wird.

Es wird durch diese Wärmerückgewinnung eine äußerst wirschaftlich arbeitende Anlage erreicht, wobei Durchsatzleistungen erzielt werden, die etwa das vierfache herkömmlicher Anlage erbringen.

## Patentansprüche

1. Anlage zum Sterilisieren, Pasteurisieren und/oder Desinfizieren pump- oder rieselfähiger Medien, in welcher zum Erhitzen ein Mikrowellengerät (1) vorgesehen ist, durch welches das zu behandelnde Medium hindurchgefördert wird, dadurch gekennzeichnet, daß im Strömungsweg zwei zwangsfördernde Förderaggregate (8, 15) vorgesehen sind, von denen eines (8) vor dem Mikrowellengerät (1) und eines nach demselben angeordnet ist, wobei zwischen den beiden Förderaggregaten (8, 15) ein Drucksensor (20) eingebaut ist, der den in der Leitung (4, 12) zwischen den beiden Förderaggregaten (8,15) herrschenden Druck als Steuersignal an eine, die beiden Förderaggregate (8, 15) miteinander logisch verbindende Steuerung (24) weiterleitet.

2. Anlage nach Anspruch 1, dadurch gekennzeichnet, daß als zwangsfördernde Förderaggregate (8, 15) zwangsfördernde Pumpen, vorzugsweise Schlauchquetschpumpen, vorgesehen sind.

3. Anlage nach Anspruch 1 oder 2, dadurch gekennzeichnet, daß dem Mikrowellengerät (1) eine Termperaturhaltestrecke (13), z.B. eine Rohrschlange nachgeschaltet ist, wobei zwischen Mikrowellengerät (1) und Temperaturhaltestrecke (13) ein Temperaturfühler (27) angeordnet ist.

4. Anlage nach Anspruch 3, dadurch gekennzeichnet, daß am Ende der Temperaturhaltestrecke (13) ein zweiter Temperaturfühler (28) angeordnet ist.

5. Anlage nach einem der Ansprüche 1 bis 4, dadurch gekennzeichnet, daß der Drucksensor (20) und der bzw. die Temperaturfühler (27, 28) mit einer zentralen Steuereinheit (24) verbunden sind, über welche die Leistung der beiden zwangsfördernden Förderaggregate (8, 15) und gegebenenfalls des Magnetrons (2) des Mikrowellengerätes (1) steuerbar ist.

6. Anlage nach einem der Ansprüche 1 bis 5, dadurch gekennzeichnet, daß das zu behandelnde Medium vor dem Mikrowellengerät (1) über einen oder mehrere Wärmetauscher (7, 5) geführt ist, in welcher das bereits behandelte Medium mit dem zu behandelnden Medium im Gegenstrom geführt ist.

7. Anlage nach Anspruch 6, dadurch gekennzeichnet, daß bei Einsatz eines wassergekühlten Magnetrons (2) das Kühlwasser durch einen weiteren Wärmetauscher (6) geführt ist, in welchem das Kühlwasser gleichfalls im Gegenstrom mit dem zu behandelnden Medium geführt ist.

8. Anlage nach einem der Ansprüche 1 bis 7, dadurch gekennzeichnet, daß innerhalb der Förderstrecke ein Abbruchgefäß (3) angeordnet ist, und zwar vorzugsweise innerhalb des Mikrowellengerätes (1).

9. Anlage nach Anspruch 8, dadurch gekennzeichnet, daß das Abbruchgefäß (3) direkt das Behandlungsgefäß für das pumpfähige Medium in dem Mikrowellengerät (1) bildet.

## Claims

1. A system for sterilising, pasteurising and/or disinfecting pumpable or sprayable media, in which a microwave apparatus (1) is provided for heating, though which the medium which is to be treated is passed, characterised in that two flow forcing appliances (8, 15) are provided in the flow path, one of these (8) being disposed before the microwave apparatus (1) and one (15) being disposed after this, wherein a pressure sensor (20) is installed between the two flow appliances (8, 15) which transmits the pressure prevalent in the pipe (4, 12) between the two conveyance appliances (8, 15) as a control signal to a control system (24) which logically connects the two flow appliances (8, 15) to one another.

2. A system according to claim 1, characterised in that two flow forcing pumps, preferably stricture pumps, are provided as flow forcing appliances (8, 15).

3. A system according to claim 1 or 2, characterised in that a temperature maintaining section (13), e.g. a coil of pipe, is included in the system after the microwave apparatus (1), wherein a temperature sensor (27) is disposed between the microwave apparatus (1) and the temperature maintaining section (13).

4. A system according to claim 3, characterised in that a second temperature sensor (28) is disposed at the end of the temperature maintaining section (13).

5. A system according to one of claims 1 to 4, characterised in that the pressure sensor (20) and the temperature sensor/s (27, 28) are connected to a central controlling unit (24) via which the performance of the two flow forcing appliances (8, 15) and if need be the magnetron (2) of the microwave apparatus (1) is controlled.

6. A system according to one of claims 1 to 5, characterised in that the medium to be treated is guided over one or more heat exchangers (7, 5) before the microwave appliance (1), the already-treated medium being guided within these heat exchangers in counter-current to the medium which is to be treated.

7. A system according to claim 6, characterised in that when using a water-cooled magnetron (2), the cooling water is guided through a further heat exchanger (6) in which the cooling water is also guided in counter-current to the medium which is to be treated.

8. A system according to one of claims 1 to 7, characterised in that a holding container (3) is disposed within the flow path, preferably within the microwave appliance (1).

9. A system according to claim 8, characterised in that the holding container (3) directly forms the treatment container for the pumpable medium in the microwave appliance (1).

## Revendications

1. Installation de stérilisation, pasteurisation et/ou désinfection de matières pompables ou susceptibles de s'écouler, dans laquelle est prévu, pour le chauffage, un dispositif à micro-ondes (1) à travers lequel la matière à traiter est amenée à circuler, caractérisée en ce que sont prévues, dans le parcours d'écoulement, deux unités de transport (8, 15) à transport forcé, dont une (8) est placée en amont du dispositif à micro-ondes (1) et une est placée en aval de celui-ci, un capteur de pression (20) étant monté entre les deux unités de transport (8, 15), capteur qui transmet, sous forme de signal de commande, la pression qui règne dans la conduite (4, 12), entre les deux unités de transport (8, 15), à une commande (24) établissant une liaison logique entre les deux unités de transport (8, 15).

2. Installation selon la revendication 1, caractérisée en ce que sont prévues, comme unités de transport (8, 15) à transport forcé, des pompes à transport forcé, de préférence des pompes péristaltiques.

3. Installation selon la revendication 1 ou 2, caractérisée en ce qu'un tronçon de maintien en température (13), par exemple un serpentin, est monté en aval du dispositif à micro-ondes (1), un capteur de température (27) étant placé entre le dispositif à microondes (1) et le tronçon de maintien en température (13).

4. Installation selon la revendication 3, caractérisée en ce qu'un deuxième capteur de température (28) est monté à l'extrémité du tronçon de maintien en température (13).

5. Installation selon l'une des revendications 1 à 4, caractérisée en ce que le capteur de pression (20) et le ou les capteur(s) de température (27, 28) sont reliés à une unité de commande centrale (24), au moyen de laquelle il est possible de commander la puissance des deux unités de transport (8, 15) à transport forcé et, éventuellement, du magnétron (2) du dispositif à micro-ondes (1).

6. Installation selon l'une des revendications 1 à 5, caractérisée en ce que la matière à traiter, présente en amont du dispositif à micro-ondes (1), est amenée à passer sur un ou plusieurs échangeurs de chaleur (7, 5), dans lesquels la matière déjà traitée circule à contre-courant par rapport à la matière à traiter.

7. Installation selon la revendication 6, caractérisée en ce que, lorsqu'est utilisé un magnétron (2) refroidi à l'eau, l'eau de refroidissement est amenée à passer à travers un autre échangeur de chaleur (6), dans lequel l'eau de refroidissement circule également, à contre-courant, par rapport à la matière à traiter.

8. Installation selon l'une des revendications 1 à 7, caractérisée en ce qu'est placé, dans le parcours de transport, une enceinte à écoulement interrompu (3), à savoir de préférence à l'intérieur du dispositif à microondes (1).

9. Installation selon la revendication 8, caractérisée en ce que l'enceinte à écoulement interrompu (3) constitue directement l'enceinte de traitement destinée à la matière pompable, à l'intérieur du dispositif à micro-ondes (1).
